(19) Europäisches Patentamt
European Patent Office
Office européen des brevets

(11) Veröffentlichungsnummer: **0 090 936**
**B1**

(12) # EUROPÄISCHE PATENTSCHRIFT

(45) Veröffentlichungstag der Patentschrift: **18.12.85**

(51) Int. Cl.⁴: **C 07 D 249/08,** C 07 D 233/60, A 01 N 43/50, A 01 N 43/64 // C07C131/00

(21) Anmeldenummer: **83101621.7**

(22) Anmeldetag: **21.02.83**

(54) **Substituierte Azolylethyl-oximinoalkyl-ether, Verfahren zu ihrer Herstellung und ihre Verwendung als Fungizide.**

(30) Priorität: **06.03.82 DE 3208194**

(43) Veröffentlichungstag der Anmeldung: **12.10.83 Patentblatt 83/41**

(45) Bekanntmachung des Hinweises auf die Patenterteilung: **18.12.85 Patentblatt 85/51**

(84) Benannte Vertragsstaaten: **AT BE CH DE FR GB IT LI NL SE**

(56) Entgegenhaltungen:
**DE - A - 1 940 388**
**DE - A - 2 613 167**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Stetter, Jörg, Dr., Gellertweg 4, D-5600 Wuppertal 1 (DE)**
Erfinder: **Büchel, Karl Heinz, Prof. Dr., Dabringhausener Strasse 42, D-5093 Burscheid (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen 1 (DE)**
Erfinder: **Frohberger, Paul-Ernst, Dr., Willi-Baumeister-Strasse 5, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 090 936 B1

ACTORUM AG

## Beschreibung

Die Anmeldung betrifft neue substituierte Azolylethyloximinoalkylether, ein Verfahren zu ihrer Herstellung sowie ihre Verwendung als Fungizide.

Es ist bereits bekannt geworden, dass bestimmte Triazolylethylether-Derivate, wie z.B. [1-(4-Chlorphenyl)-2-(1,2,4-triazol-1-yl)]ethylallylether und [1-(2,4-Dichlorphenyl)-2-(1,2,4-triazol-1-yl)]ethyl-n-butylether, allgemein gute fungizide Eigenschaften besitzen (vergleiche DE-OS Nr. 2547953). Die Wirkung dieser Verbindungen ist jedoch in bestimmten Indikationsbereichen, insbesondere bei niedrigen Aufwandmengen und -konzentrationen, nicht immer ganz befriedigend.

Es wurden neue substituierte Azolylethyloximinoalkylether der allgemeinen Formel

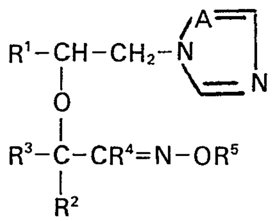

(I)

in welcher:

A für ein Stickstoffatom oder die CH-Gruppe steht,

$R^1$ für ggf. einfach oder zweifach, gleich oder verschieden substituiertes Phenyl steht, wobei als Substituenten Halogen, Alkyl mit 1 bis 4 Kohlenstoffatomen, Alkoxy mit 1 bis 2 Kohlenstoffatomen sowie Halogenalkyl und Halogenalkoxy mit jeweils 1 bis 2 Kohlenstoff- und 1 bis 5 gleichen oder verschiedenen Halogenatomen zu nennen sind,

$R^2$ für Wasserstoff, für Alkyl mit 1 bis 4 Kohlenstoffatomen sowie für ggf. einfach oder zweifach, gleich oder verschieden substituiertes Phenyl steht, wobei als Substituenten die bei $R^1$ genannten Phenylsubstituenten in Frage kommen,

$R^3$ für Wasserstoff, für Alkyl mit 1 bis 4 Kohlenstoffatomen sowie für ggf. einfach oder zweifach gleich oder verschieden substituiertes Phenyl steht, wobei als Substituenten die bei $R^1$ genannten Phenylsubstituenten in Frage kommen,

$R^4$ für Wasserstoff, für Alkyl mit 1 bis 4 Kohlenstoffatomen sowie für ggf. einfach oder zweifach, gleich oder verschieden substituiertes Phenyl steht, wobei als Substituenten die bei $R^1$ genannten Phenylsubstituenten in Frage kommen, und

$R^5$ für Wasserstoff, für Alkyl mit 1 bis 4 Kohlenstoffatomen, Alkenyl und Alkinyl mit jeweils 2 bis 4 Kohlenstoffatomen, sowie für jeweils ggf. einfach oder zweifach, gleich oder verschieden substituiertes Phenyl und Phenylalkyl mit 1 bis 2 Kohlenstoffatomen im Alkylteil steht, wobei als jeweilige Substituenten an den aromatischen Resten die bei $R^1$ genannten Phenylsubstituenten in Frage kommen,

und deren Säureadditions-Salze und Metallsalz-Komplexe gefunden.

Die Verbindungen der Formel I können in der syn- oder anti-Form vorliegen; vorwiegend fallen sie als Gemische beider Formen an.

Weiterhin wurde gefunden, dass man die substituierten Azolylethyloximinoalkylether der Formel I sowie deren Säureadditions-Salze und Metallsalz-Komplexe erhält, wenn man Azolylethanole der Formel

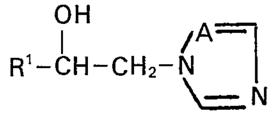

(II)

in welcher:

A und $R^1$ die oben angegebene Bedeutung haben,

mit Oxim-Derivaten der Formel

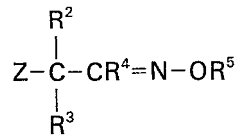

(III)

in welcher:

$R^2$, $R^3$, $R^4$ und $R^5$ die oben angegebene Bedeutung haben, und

Z für Halogen, den Methylsulfonyloxy- oder den Tolylsulfonyloxy-Rest steht,

in Gegenwart einer Base und in Gegenwart eines organischen Verdünnungsmittels, oder in einem wässerig-organischen Zweiphasensystem in Gegenwart eines Phasentransferkatalysators, umsetzt, und ggf. anschliessend eine Säure oder ein Metallsalz addiert.

Die neuen substituierten Azolylethyloximinoalkylether der Formel I weisen starke fungizide Eigenschaften auf. Dabei zeigen überraschenderweise die erfindungsgemässen Verbindungen eine bessere fungizide Wirkung als die aus dem Stand der Technik bekannten Triazolylethylether-Derivate, wie z.B. [1-(4-Chlorphenyl)-2-(1,2,4-triazol-1-yl)]ethylallylether und [1-(2,4-Dichlorphenyl)-2-(1,2,4-triazol-1-yl)]ethyl-n-butylether, die chemisch und wirkungsmässig naheliegende Verbindungen sind. Die erfindungsgemässen Stoffe stellen somit eine Bereicherung der Technik dar.

Die erfindungsgemässen substituierten Azolylethyloximinoalkylether sind durch die Formel I allgemein definiert.

Bevorzugt sind diejenigen Verbindungen der Formel I, in denen:

A für ein Stickstoffatom oder die CH-Gruppe steht;

$R^1$ für ggf. einfach oder zweifach, gleich oder verschieden substituiertes Phenyl steht, wobei als Substituenten insbesondere genannt seien: Fluor, Chlor, Brom, Methyl, Ethyl, Methoxy, Trifluormethyl und Trifluormethoxy;

$R^2$ für Wasserstoff, Methyl, Ethyl, Isopropyl sowie für ggf. einfach oder zweifach, gleich oder verschieden substituiertes Phenyl steht, wobei als Substituenten insbesondere die für $R^1$ bereits genannten Phenylsubstituenten in Frage kommen;

$R^3$ für Wasserstoff, Methyl, Ethyl, Isopropyl sowie für ggf. einfach oder zweifach, gleich oder verschieden substituiertes Phenyl steht, wobei als Substituenten insbesondere die für $R^1$ bereits genannten Phenylsubstituenten in Frage kommen;

$R^4$ für Wasserstoff, Methyl, Ethyl, Isopropyl sowie für ggf. einfach oder zweifach, gleich oder verschieden substituiertes Phenyl steht, wobei als Substituenten insbesondere die für $R^1$ bereits genannten Phenylsubstituenten in Frage kommen, und

$R^5$ für Wasserstoff, Methyl, Ethyl, Isopropyl, n-Propyl, n-Butyl, sek.-Butyl, tert.-Butyl, Vinyl, Allyl, Propargyl sowie für jeweils ggf. einfach oder zweifach, gleich oder verschieden substituiertes Phenyl und Benzyl steht, wobei als Substituenten insbesondere die für $R^1$ bereits genannten Phenylsubstituenten in Frage kommen.

Bevorzugte erfindungsgemässe Verbindungen sind auch Additionsprodukte aus Säuren und denjenigen substituierten Azolylethyloximinoalkylethern der Formel I, in denen die Substituenten A, $R^1$, $R^2$, $R^3$, $R^4$ und $R^5$ die Bedeutungen haben, die bereits vorzugsweise für diese Reste genannt wurden.

Zu den Säuren, die addiert werden können, gehören vorzugsweise Halogenwasserstoffsäuren, wie z. B. die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Salicylsäure, Sorbinsäure und Milchsäure, sowie Sulfonsäuren, wie z. B. p-Toluolsulfonsäure und 1,5-Naphthalindisulfonsäure.

Ausserdem bevorzugte erfindungsgemässe Verbindungen sind Additionsprodukte aus Salzen von Metallen der II. bis IV. Haupt- und der I. und II. sowie IV. bis VIII. Nebengruppe und denjenigen substituierten Azolylethyloximinoalkylethern der Formel I, in denen die Substituenten A, $R^1$, $R^2$, $R^3$, $R^4$ und $R^5$ die Bedeutungen haben, die bereits vorzugsweise für diese Reste genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure.

Verwendet man beispielsweise 1-(2,4-Dichlorphenyl)-2-(1,2,4-triazol-1-yl)ethanol und 1-Chlor-2-methoximino-2-phenylethan als Ausgangsstoffe, so kann der Reaktionsablauf des erfindungsgemässen Verfahrens durch das folgende Formelschema wiedergegeben werden:

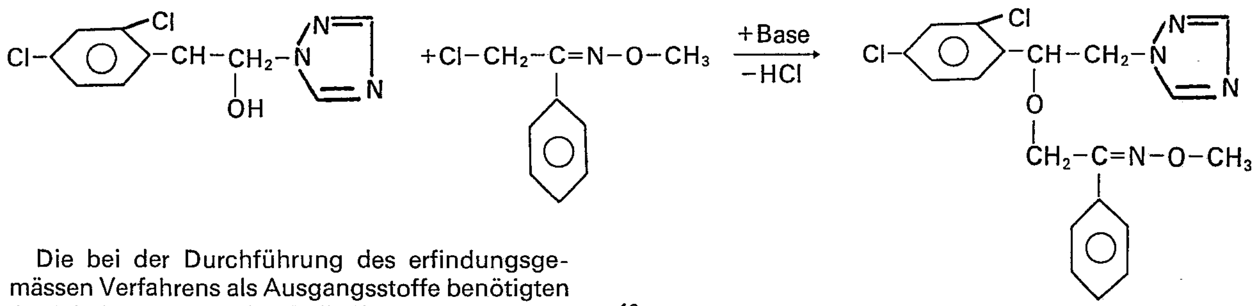


Die bei der Durchführung des erfindungsgemässen Verfahrens als Ausgangsstoffe benötigten Azolylethanole sind durch die Formel II allgemein definiert. In dieser Formel stehen A und $R^1$ vorzugsweise für die Bedeutungen, die bei der Beschreibung der erfindungsgemässen Stoffe der Formel I bereits vorzugsweise genannt wurden.

Die Azolylethanole der Formel II sind bekannt (vergleiche z. B. DE-OS Nrn. 2431407, 2638470 und 1940388); bzw. können sie in allgemein bekannter Art und Weise erhalten werden, indem man die entsprechenden α-Brom(Chlor)ketone mit Imidazol oder 1,2,4-Triazol in Gegenwart eines inerten organischen Lösungsmittels, wie beispielsweise Aceton, und in Gegenwart eines Säurebindemittels, wie beispielsweise Kaliumcarbonat, vorzugsweise in der Siedehitze umsetzt und die so erhaltenen Azolylethanone in üblicher Weise mit komplexen Hydriden, wie beispielsweise Natriumborhydrid, oder mit Aluminiumisopropylat reduziert.

Die ausserdem für das erfindungsgemässe Verfahren als Ausgangsstoffe zu verwendenden Oxim-Derivate sind durch die Formel III allgemein definiert. In dieser Formel stehen $R^2$, $R^3$, $R^4$ und $R^5$ vorzugsweise für die Bedeutungen, die bei der Beschreibung der erfindungsgemässen Stoffe der Formel I bereits vorzugsweise genannt wurden. Z steht vorzugsweise für Chlor, Brom, den Methylsulfonyloxy- und den Tolylsulfonyloxy-Rest.

Die Oxim-Derivate der Formel III sind bekannt (vergleiche z. B. US-PS Nr. 3896189 und DE-OS Nr. 2922759); bzw. können sie in allgemein bekannter Art und Weise erhalten werden, indem man entsprechende Carbonylverbindungen mit Hydroxylamin-(Derivaten) in Gegenwart eines Lösungsmittels, vorzugsweise eines Alkohols, bei Temperaturen zwischen 20 und 100° C, vorzugsweise zwischen 50 und 80° C umsetzt. Dabei wird das Hydroxylamin-(Derivat) vorzugsweise in Form eines Salzes, insbesondere als Hydrochlorid, ggf. in Gegenwart eines Säurebindemittels, wie beispielsweise Natriumacetat, eingesetzt. Die Isolierung der Endprodukte erfolgt in üblicher Weise. Einzelne Oxim-Derivate der Formel III können auch erhalten werden, wenn man Oximether der Formel

$$R^3-CH_2-CR^4=N-OR^5 \qquad (IV)$$

in welcher:

$R^3$, $R^4$ und $R^5$ die oben angegebene Bedeutung haben,

in üblicher Weise halogeniert [vergleiche hierzu «J. Org. Chem.», *36* (1971) 3467].

Als Verdünnungsmittel kommen für das erfindungsgemässe Verfahren vorzugsweise inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Als Basen können für das erfindungsgemässe Verfahren alle üblichen organischen und anorganischen Basen eingesetzt werden. Hierzu gehören vorzugsweise tertiäre Amine, beispielsweise Triethylamin oder Pyridin und Alkalihydroxide oder Alkalicarbonate, beispielsweise Natrium- und Kaliumhydroxid, sowie Alkalimetallhydride wie beispielsweise Natriumhydrid.

Die Reaktionstemperaturen können bei der Durchführung des erfindungsgemässen Verfahrens in einem grösseren Bereich variiert werden. Im allgemeinen arbeitet man zwischen 0 und 150° C, vorzugsweise zwischen 20 und 100° C.

Bei der Durchführung des erfindungsgemässen Verfahrens arbeitet man vorzugsweise mit äquimolaren Mengen. Es ist jedoch auch möglich, eine der Komponenten in einem Überschuss einzusetzen. Die Aufarbeitung und Isolierung der Reaktionsprodukte erfolgt nach üblichen Methoden.

In einer bevorzugten Ausführungsform wird die erfindungsgemässe Umsetzung in einem Zweiphasensystem, wie beispielsweise wässerige Natron- oder Kalilauge/Toluol oder Methylenchlorid durchgeführt, ggf. unter Zusatz von 0,1-1 mol eines Phasentransfer-Katalysators, wie beispielsweise einer Ammonium- oder Phosphoniumverbindung, beispielsweise seien Benzyldodecyldimethylammoniumchlorid und Triethylbenzylammoniumchlorid genannt.

Zur Herstellung von Säureadditions-Salzen der Verbindungen der Formel I kommen vorzugsweise diejenigen Säuren in Frage, die bereits im Zusammenhang mit der Beschreibung der erfindungsgemässen Säureadditions-Salze als bevorzugte Säuren genannt wurden.

Die Säureadditions-Salze der Verbindungen der Formel I können in einfacher Weise nach üblichen Salzbildungsmethoden, durch Lösen einer Verbindung der Formel I in einem geeigneten Lösungsmittel und Hinzufügen der Säure, z.B. Chlorwasserstoffsäure, erhalten werden und in bekannter Weise, z. B. durch Abfiltrieren, isoliert und ggf. durch Waschen mit einem inerten organischen Lösungsmittel gereinigt werden.

Zur Herstellung von Metallsalz-Komplexen von Verbindungen der Formel I kommen vorzugsweise Salze von denjenigen Anionen und Kationen in Betracht, die bereits im Zusammenhang mit der Beschreibung der erfindungsgemässen Metallsalz-Komplexe als bevorzugt genannt wurden.

Die Metallsalz-Komplexe von Verbindungen der Formel I können in einfacher Weise nach üblichen Verfahren erhalten werden, so z.B. durch Lösen des Metallsalzes in Alkohol, z.B. Ethanol, und Hinzufügen der Lösung zur Verbindung der Formel I. Man kann Metallsalz-Komplexe in bekannter Weise, z.B. durch Abfiltrieren, isolieren und ggf. durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophormycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gerstenmehltaus (Erysiphe graminis), von Leptosphaeria-Arten, wie z.B. gegen den Erreger der Braunfleckenkrankheit an Weizen (Leptosphaeria nodorum), von Sphaerotheca-Arten, wie z.B. gegen den Erreger des Gurkenmehltaus (Sphaerotheca fuliginea), sowie von Podosphaera-Arten, wie z.B. gegen den Erreger des Apfelmehltaus (Podosphaera leucotricha); ausserdem auch zu Bekämpfung von Reiskrankheiten, wie z.B. Pyricularia oryzae und Pellicularia sasakii.

In entsprechenden Aufwandmengen zeigen die erfindungsgemässen Stoffe auch herbizide Wirksamkeit.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, ggf. unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglykolether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe, wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe, wie Salze von Eisen, Mangan, Bor, Kupfer, Kobalt, Molybdän und Zink, verwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trokkenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

*Herstellungsbeispiele*

*Beispiel 1:*

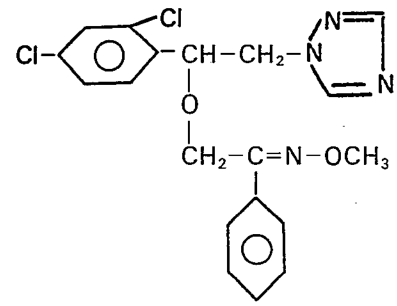

12,9 g (0,05 mol) 1-(2,4-Dichlorphenyl)-2-(1,2,4-triazol-1-yl)ethanol werden in einem Zweiphasensystem aus 150 ml Methylenchlorid und 30 ml 50%iger Natronlauge unter Zusatz von 150 g Triethylbenzylammoniumchlorid heftig gerührt und tropfenweise mit 11 g (0,06 mol) 1-Chlor-2-methoximino-2-phenylethan versetzt. Man lässt das Reaktionsgemisch 6 h bei 42° C rühren, trennt die organische Phase ab, wäscht sie mit Wasser neutral und trocknet über Natriumsulfat. Anschliessend wird das Lösungsmittel im Vakuum abgetrennt und der Rückstand im Hochvakuum entgast. Nach Verreiben mit Petrolether kristallisiert der Rückstand aus. Man erhält 8,2 g (41% der Theorie) 1-(2,4-Dichlorphenyl)-1-(2-methoximino-2-phenylethoxy)ethan vom Schmelzpunkt 82-84° C.

*Herstellung des Ausgangsproduktes*

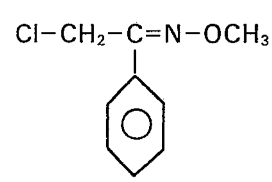

46,3 g (0,3 mol) ω-Chloracetophenon, 27,5 g (0,33 mol) O-Methylhydroxylaminhydrochlorid und 27 g (0,33 mol) Natriumacetat werden 3 h in

Ethanol unter Rückfluss erhitzt. Anschliessend wird der anorganische Niederschlag abfiltriert und das Filtrat eingeengt. Der Rückstand wird zwischen Wasser und Methylenchlorid verteilt, die organische Phase wird abgetrennt, über Natriumsulfat getrocknet und eingeengt. Der Rückstand wird im Hochvakuum destilliert. Man erhält 44 g (80% der Theorie) 1-Chlor-2-methoximino-2-phenylethan vom Siedepunkt 65° C/0,3 mbar.

In entsprechender Weise und gemäss dem erfindungsgemässen Verfahren werden die Verbindungen der folgenden Tabelle der allgemeinen Formel

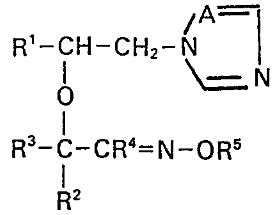

(I)

erhalten:

| Bsp. Nr. | $R^1$ | $R^2$ | $R^3$ | $R^4$ | $R^5$ | A | Physikal. Konstante |
|---|---|---|---|---|---|---|---|
| 2 | 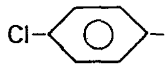 | H | H | H | $CH_3$ | N | Kp: 180° C/0,1 mbar |
| 3 | 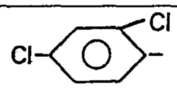 | H | H | H | $CH_3$ | N | $n_D^{20}$: 1,5555 |
| 4 | 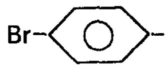 | H | H | H | $CH_3$ | N | Kp: 190° C/0,1 mbar |
| 5 | 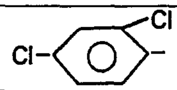 | H | $CH_3$ | H | $CH_3$ | N | Fp: 90-113° C |
| 6 | 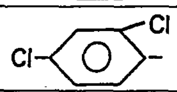 | H | H | $CH_3$ | $CH_3$ | N | Kp: 190° C/0,2mbar |
| 7 | 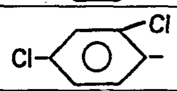 | H | $CH_3$ | $CH_3$ | $CH_3$ | N | zähes Öl |
| 8 | 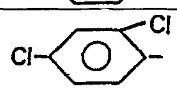 | H | H | H | $CH_3$ | CH | Kp: 170° C/0,2mbar |
| 9 | 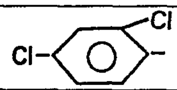 | H | $CH_3$ | H | $CH_3$ | CH | Kp: 160° C/0,1 mbar |
| 10 | 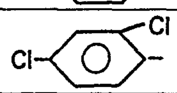 | H | H | $CH_3$ | $CH_3$ | CH | zähes Öl |
| 11 | 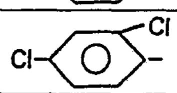 | H | H | 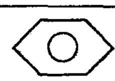 | $CH_3$ | CH | zähes Öl |
| 12 | 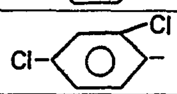 | H | H | 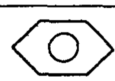 | $CH_3$ | CH | Fp: 186-88° C (× ½ NDS)* |
| 13 | 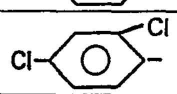 | H | $CH_3$ | $CH_3$ | $CH_3$ | CH | zähes Öl |
| 14 | 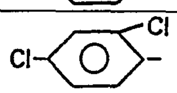 | H | $CH_3$ | H | $C_2H_5$ | CH | Kp: 170° C/0,2mbar |
| 15 | 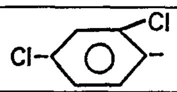 | H | H | 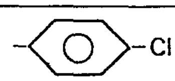 | $CH_3$ | N | Fp: 78° C |
| 16 | 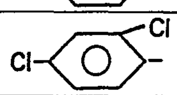 | H | H | 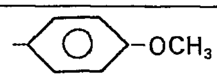 | $CH_3$ | N | zähes Öl |
| 17 | 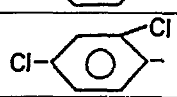 | H | H | 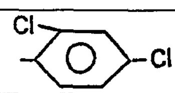 | $CH_3$ | N | $n_D^{20}$=1,5864 |
| 18 | 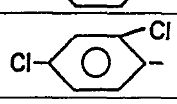 | H | H | 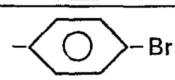 | $CH_3$ | N | Fp: 128-30°C |

* NDS = 1,5-Naphthalindisulfonsäure.

*Verwendungsbeispiele:*

In den nachfolgenden Beispielen werden die nachstehend angegebenen Verbindungen als Vergleichssubstanzen eingesetzt:

(A) 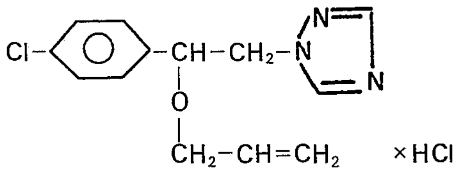

(B) 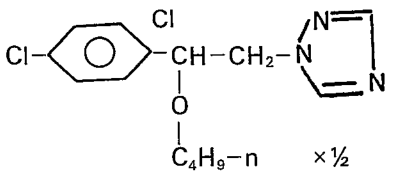

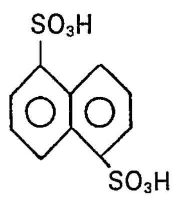

*Beispiel A:*

*Erysiphe-Test (Gerste)/protektiv*

Lösungsmittel: 100 Gew.-Teile Dimethylformamid

Emulgator: 0,25 Gew.-Teile Alkylarylpolyglykolether

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20° C und einer relativen Luftfeuchtigkeit von ca. 80% aufgestellt, um die Entwicklung von Mehltaupusteln zu begünstigen.

7 d nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 3, 4, 8, 9 und 6.

*Beispiel B:*

*Erysiphe-Test (Gerste)/Saatgutbehandlung*

Die Anwendung der Wirkstoffe erfolgt als Trokkenbeizmittel. Sie werden zubereitet durch Abstrecken des jeweiligen Wirkstoffes mit Gesteinsmehl zu einer feinpulvrigen Mischung, die eine gleichmässige Verteilung auf der Saatgutoberfläche gewährleistet.

Zur Beizung schüttelt man das Saatgut 3 min lang mit dem Beizmittel in einer verschlossenen Glasflasche.

Die Gerste sät man mit 3 × 12 Korn 2 cm tief in eine Standarderde. 7 d nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20° C und einer relativen Luftfeuchtigkeit von ca. 80% aufgestellt, um die Entwicklung von Mehltaupusteln zu begünstigen.

7 d nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 3 und 8.

*Beispiel C:*

*Leptosphaeria nodorum-Test (Weizen)/protektiv*

Lösungsmittel: 100 Gew.-Teile Dimethylformamid

Emulgator: 0,25 Gew.-Teile Alkylarylpolyglykolether

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 h bei 20° C und 100% relativer Luftfeuchtigkeit in einer Inkubationskabine.

Die Pflanzen werden in einem Gewächshaus bei einer Temperatur von ca. 15° C und einer relativen Luftfeuchtigkeit von ca. 80% aufgestellt.

10 d nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 8 und 9.

*Beispiel D:*

*Podosphaera-Test (Apfel)/protektiv*

Lösungsmittel: 4,7 Gew.-Teile Aceton

Emulgator: 0,3 Gew.-Teile Alkylarylpolyglykolether

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23° C und einer relativen Luftfeuchtigkeit von ca. 70% aufgestellt.

9 d nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 3, 2, 4, 8, 9, 6 und 10.

*Beispiel E:*

*Sphaerotheca-Test (Gurke)/protektiv*

Lösungsmittel: 4,7 Gew.-Teile Aceton

Emulgator: 0,3 Gew.-Teile Alkylarylpolyglykolether

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschliessend bei 23 bis 24° C und bei einer relativen Luftfeuchtigkeit von ca. 75% im Gewächshaus aufgestellt.

10 d nach der Inokulation erfolgt die Auswertung.

Eine deutliche Überlegenheit in der Wirksamkeit gegenüber dem Stand der Technik zeigen in diesem Test z.B. die Verbindungen gemäss folgender Herstellungsbeispiele: 3, 9 und 8.

## Patentansprüche

1. Substituierte Azolylethyloximinoalkylether der allgemeinen Formel I

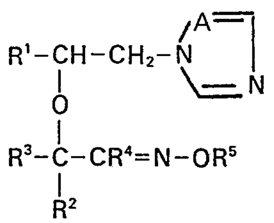

(I)

in welcher:

A für ein Stickstoffatom oder die CH-Gruppe steht,

$R^1$ für ggf. einfach oder zweifach, gleich oder verschieden substituiertes Phenyl steht, wobei als Substituenten Halogen, Alkyl mit 1 bis 4 Kohlenstoffatomen, Alkoxy mit 1 bis 2 Kohlenstoffatomen sowie Halogenalkyl und Halogenalkoxy mit jeweils 1 bis 2 Kohlenstoff- und 1 bis 5 gleichen oder verschiedenen Halogenatomen zu nennen sind,

$R^2$ für Wasserstoff, für Alkyl mit 1 bis 4 Kohlenstoffatomen sowie für ggf. einfach oder zweifach, gleich oder verschieden substituiertes Phenyl steht, wobei als Substituenten die bei $R^1$ genannten Phenylsubstituenten in Frage kommen,

$R^3$ für Wasserstoff, für Alkyl mit 1 bis 4 Kohlenstoffatomen sowie für ggf. einfach oder zweifach; gleich oder verschieden substituiertes Phenyl steht, wobei als Substituenten die bei $R^1$ genannten Phenylsubstituenten in Frage kommen,

$R^4$ für Wasserstoff, für Alkyl mit 1 bis 4 Kohlenstoffatomen sowie für ggf. einfach oder zweifach, gleich oder verschieden substituiertes Phenyl steht, wobei als Substituenten die bei $R^1$ genannten Phenylsubstituenten in Frage kommen, und

$R^5$ für Wasserstoff, für Alkyl mit 1 bis 4 Kohlenstoffatomen, Alkenyl und Alkinyl mit jeweils 2 bis 4 Kohlenstoffatomen sowie für jeweils ggf. einfach oder zweifach, gleich oder verschieden substituiertes Phenyl und Phenylalkyl mit 1 bis 2 Kohlenstoffatomen im Alkylteil steht, wobei als jeweilige Substituenten an den aromatischen Resten die bei $R^1$ genannten Phenylsubstituenten in Frage kommen,

und deren Säureadditions-Salze und Metallsalz-Komplexe.

2. Verbindungen der allgemeinen Formel I in Anspruch 1, wobei:

A für ein Stickstoffatom oder die CH-Gruppe steht;

$R^1$ für ggf. einfach oder zweifach, gleich oder verschieden durch Fluor, Chlor, Brom, Methyl, Ethyl, Methoxy, Trifluormethyl und Trifluormethoxy substituiertes Phenyl steht;

$R^2$ für Wasserstoff, Methyl, Ethyl, Isopropyl sowie für ggf. einfach oder zweifach, gleich oder verschieden durch die für $R^1$ bereits genannten Phenylsubstituenten substituiertes Phenyl steht;

$R^3$ für Wasserstoff, Methyl, Ethyl, Isopropyl sowie für ggf. einfach oder zweifach, gleich oder verschieden durch die für $R^1$ bereits genannten Phenylsubstituenten substituiertes Phenyl steht;

$R^4$ für Wasserstoff, Methyl, Ethyl, Isopropyl sowie für ggf. einfach oder zweifach, gleich oder verschieden durch die für $R^1$ bereits genannten Phenylsubstituenten substituiertes Phenyl steht;

$R^5$ für Wasserstoff, Methyl, Ethyl, Isopropyl, n-Propyl, n-Butyl, sek.-Butyl, tert.-Butyl, Vinyl, Allyl, Propargyl sowie für jeweils ggf. einfach oder zweifach, gleich oder verschieden durch die für $R^1$ bereits genannten Phenylsubstituenten substituiertes Phenyl und Benzyl steht;

und deren Säureadditions-Salze und Metallsalz-Komplexe.

3. Verfahren zur Herstellung von substituierten Azolylethyloximinoalkylethern der allgemeinen Formel I

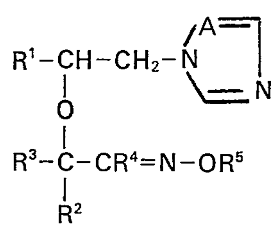

(I)

in welcher:

A für ein Stickstoffatom oder die CH-Gruppe steht,

$R^1$ für ggf. einfach oder zweifach, gleich oder verschieden substituiertes Phenyl steht, wobei als Substituenten Halogen, Alkyl mit 1 bis 4 Kohlenstoffatomen, Alkoxy mit 1 bis 2 Kohlenstoffatomen sowie Halogenalkyl und Halogenalkoxy mit jeweils 1 bis 2 Kohlenstoff- und 1 bis 5 gleichen oder verschiedenen Halogenatomen zu nennen sind,

$R^2$ für Wasserstoff, für Alkyl mit 1 bis 4 Kohlenstoffatomen sowie für ggf. einfach oder zweifach, gleich oder verschieden substituiertes Phenyl steht, wobei als Substituenten die bei $R^1$ genannten Phenylsubstituenten in Frage kommen,

$R^3$ für Wasserstoff, für Alkyl mit 1 bis 4 Kohlenstoffatomen sowie für ggf. einfach oder zweifach, gleich oder verschieden substituiertes Phenyl steht, wobei als Substituenten die bei $R^1$ genannten Phenylsubstituenten in Frage kommen,

$R^4$ für Wasserstoff, für Alkyl mit 1 bis 4 Kohlenstoffatomen sowie für ggf. einfach oder zweifach, gleich oder verschieden substituiertes Phenyl steht, wobei als Substituenten die bei $R^1$ genannten Phenylsubstituenten in Frage kommen, und

$R^5$ für Wasserstoff, für Alkyl mit 1 bis 4 Kohlenstoffatomen, Alkenyl und Alkinyl mit jeweils 2 bis 4 Kohlenstoffatomen sowie für jeweils ggf. einfach oder zweifach, gleich oder verschieden substituiertes Phenyl und Phenylalkyl mit 1 bis 2 Kohlenstoffatomen im Alkylteil steht, wobei als jeweilige Substituenten an den aromatischen Resten die bei $R^1$ genannten Phenylsubstituenten in Frage kommen,

dadurch gekennzeichnet, dass man Azolylethanole der allgemeinen Formel II

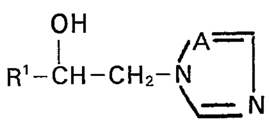

(II)

in welcher:

A und $R^1$ die oben angegebene Bedeutung haben,

mit Oxim-Derivaten der allgemeinen Formel III

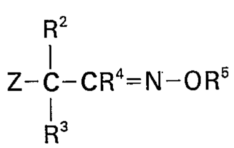

(III)

in welcher:

$R^2$, $R^3$, $R^4$ und $R^5$ die oben angegebene Bedeutung haben, und

Z für Halogen, den Methylsulfonyloxy- oder den Tolylsulfonyloxy-Rest steht,

in Gegenwart einer Base und in Gegenwart eines organischen Verdünnungsmittels, oder in einem wässerig-organischen Zweiphasensystem in Gegenwart eines Phasentransferkatalysators, umsetzt, und ggf. anschliessend eine Säure oder ein Metallsalz addiert.

4. Fungizide Mittel, gekennzeichnet durch einen Gehalt an mindestens einem substituierten Azolylethyloximinoalkylether der allgemeinen Formel I in Ansprüchen 1 und 3.

5. Verwendung von substituierten Azolylethyloximinoalkylethern der allgemeinen Formel I in Ansprüchen 1 und 3 zur Bekämpfung von Pilzen.

**Claims**

1. Substituted azolylethyl oximinoalkyl ethers of the general Formula I:

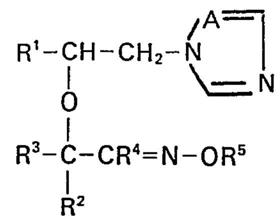

(I)

in which:

A represents a nitrogen atom or the CH group,

$R^1$ represents phenyl which is optionally mono- or disubstituted by identical or different substituents, those to be mentioned being: halogen, alkyl with 1 to 4 carbon atoms, alkoxy with 1 to 2 carbon atoms and halogenoalkyl and halogenoalkoxy each with 1 to 2 carbon and 1 to 5 identical or different halogen atoms,

$R^2$ represents hydrogen, alkyl with 1 to 4 carbon atoms and phenyl which is optionally mono- or disubstituted by identical or different substituents, possible substituents being the phenyl substituents mentioned under $R^1$,

$R^3$ represents hydrogen, alkyl with 1 to 4 carbon atoms and phenyl which is optionally mono- or disubstituted by identical or different substituents, possible substituents being the phenyl substituents mentioned under $R^1$,

$R^4$ represents hydrogen, alkyl with 1 to 4 carbon atoms and phenyl which is optionally mono- or disubstituted by identical or different substituents, possible substituents being the phenyl substituents mentioned under $R^1$, and

$R^5$ represents hydrogen, alkyl with 1 to 4 carbon atoms, alkenyl and alkinyl each with 2 to 4 carbon atoms, and phenyl and phenylalkyl with 1 to 2 carbon atoms in the alkyl part, each of which is optionally mono- or disubstituted by identical or different substituents, the possible respective substituents on the aromatic radicals being the phenyl substituents mentioned under $R^1$,

and acid addition salts and metal salt complexes thereof.

2. Compounds of the general Formula I in Claim 1, wherein:

A represents a nitrogen atom or the CH Group;

$R^1$ represents phenyl which is optionally mono- or disubstituted by identical or different substituents from amongst fluorine, chlorine, bromine, methyl, ethyl, methoxy, trifluoromethyl and trifluoromethoxy;

$R^2$ represents hydrogen, methyl, ethyl, isopropyl and phenyl which is optionally mono- or disubstituted by identical or different substituents from amongst the phenyl substituents already mentioned for $R^1$;

$R^3$ represents hydrogen, methyl, ethyl, isopropyl and phenyl which is optionally mono- or disubstituted by identical or different substituents from amongst the phenyl substituents already mentioned for $R^1$;

$R^4$ represents hydrogen, methyl, ethyl, isopropyl and phenyl which is optionally mono- or disubstituted by identical or different substituents from amongst the phenyl substituents already mentioned for $R^1$;

$R^5$ represents hydrogen, methyl, ethyl, isopropyl, n-propyl, n-butyl, sec.-butyl, tert.-butyl, vinyl, allyl, propargyl and phenyl and benzyl, each of which is optionally mono- or disubstituted by identical or different substituents from amongst the phenyl substituents already mentioned for $R^1$; and acid addition salts and metal salt complexes thereof.

3. Process for the preparation of substituted azolylethyl oximinoalkyl ethers of the general Formula I:

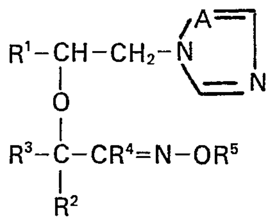

(I)

in which:

A represents a nitrogen atom or the CH group,

$R^1$ represents phenyl which is optionally mono- or disubstituted by identical or different substituents, those to be mentioned being: halogen, alkyl with 1 to 4 carbon atoms, alkoxy with 1 to 2 carbon atoms and halogenoalkyl and halogenoalkoxy each with 1 to 2 carbon and 1 to 5 identical or different halogen atoms,

$R^2$ represents hydrogen, alkyl with 1 to 4 carbon atoms and phenyl which is optionally mono- or disubstituted by identical or different substituents, possible substituents being the phenyl substituents mentioned under $R^1$,

$R^3$ represents hydrogen, alkyl with 1 to 4 carbon atoms and phenyl which is optionally mono- or disubstituted by identical or different substituents, possible substituents being the phenyl substituents mentioned under $R^1$,

$R^4$ represents hydrogen, alkyl with 1 to 4 carbon atoms and phenyl which is optionally mono- or disubstituted by identical or different substituents, possible substituents being the phenyl substituents mentioned under $R^1$, and

$R^5$ represents hydrogen, alkyl with 1 to 4 carbon atoms, alkenyl and alkinyl each with 2 to 4 carbon atoms, and phenyl and phenylalkyl with 1 to 2 carbon atoms in the alkyl part, each of which is optionally mono- or disubstituted by identical or different substituents, the possible respective substituents on the aromatic radicals being the phenyl substituents mentioned under $R^1$,

characterised in that azolyl ethanols of the general Formula II:

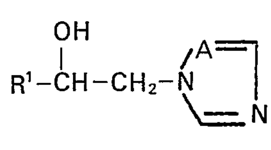

(II)

in which:

A and $R^1$ have the above mentioned meaning, are reacted with oxime derivatives of the general Formula III:

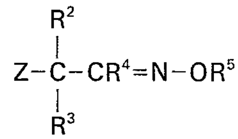

(III)

in which:

$R^2$, $R^3$, $R^4$ and $R^5$ have the above mentioned meaning, and

Z represents halogen, the methylsulphonyloxy or the tolylsulphonyloxy radical,

in the presence of a base and in the presence of an organic diluent, or in an aqueous-organic two-phase system in the presence of a phase transfer catalyst; and, if desired, an acid or a metal salt is then added on.

4. Fungicidal agents, characterised in that they contain at least one substituted azolylethyl oximinoalkyl ether of the general Formula I in Claims 1 and 3.

5. Use of substituted azolylethyl oximinoalkyl ethers of the general Formula I in Claims 1 and 3 for combating fungi.

## Revendications

1. Azolyléthyloximinoalcoyléthers substitués de formule générale:

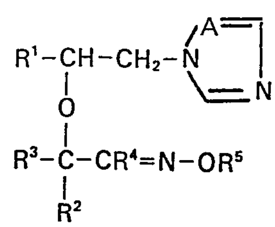

(I)

dans laquelle:

A représente un atome d'azote ou le groupe CH,

$R^1$ un phényle éventuellement substitué une ou deux fois, de manière identique ou différente, en citant comme substituants de l'halogène, alcoyle ayant 1 à 4 atomes de carbone, alcoxy ayant 1 à 2 atomes de carbone, de même qu'halogéno-alcoyle et halogénoalcoxy ayant chacun 1 à 2 atomes de carbone et 1 à 5 atomes d'halogène identiques ou différents,

$R^2$ de l'hydrogène, un alcoyle ayant 1 à 4 atomes de carbone, de même qu'un phényle éventuellement substitué une ou deux fois, de manière identique ou différente, en envisageant comme substituants les substituants de phényle cités pour $R^1$,

$R^3$ de l'hydrogène, un alcoyle ayant 1 à 4 atomes de carbone, de même qu'un phényle éventuellement substitué une ou deux fois de manière identique ou différente, en envisageant comme substituants les substituants de phényle cités pour $R^1$,

$R^4$ de l'hydrogène, un alcoyle ayant 1 à 4 atomes de carbone, de même qu'un phényle éventuellement substitué une ou deux fois de manière identique ou différente, en envisageant comme substituants les substituants de phényle cités pour $R^1$, et

$R^5$ de l'hydrogène, un alcoyle ayant 1 à 4 atomes de carbone, un alcényle et un alcinyle ayant chacun 2 à 4 atomes de carbone, de même qu'un phényle et phénylalcoyle éventuellement substitués une ou deux fois de manière identique ou différente, avec 1 à 2 atomes de carbone dans la portion alcoyle, en envisageant comme substituants chaque fois sur les radicaux aromatiques les substituants de phényle cités pour $R^1$, et

leurs sels d'addition d'acides et complexes de sels métalliques.

2. Composés de formule générale I selon la revendication 1, où

A représente un atome d'azote ou le groupe CH,

$R^1$ un phényle substitué éventuellement une ou deux fois de manière identique ou différente par du fluor, chlore, brome, méthyle, éthyle, méthoxy, trifluorométhyle et trifluorométhoxy,

$R^2$ de l'hydrogène, méthyle, éthyle, isopropyle de même qu'un phényle substitué éventuellement une ou deux fois de manière identique ou différente par les substituants de phényle déjà cités pour $R^1$,

$R^3$ de l'hydrogène, méthyle, éthyle, isopropyle de même qu'un phényle substitué éventuellement une ou deux fois, de manière identique ou différente, par les substituants de phényle déjà cités pour $R^1$,

$R^4$ de l'hydrogène, méthyle, éthyle, isopropyle, de même qu'un phényle substitué éventuellement une ou deux fois de manière identique ou différente par les substituants de phényle déjà cités pour $R^1$,

$R^5$ de l'hydrogène, méthyle, éthyle, isopropyle, n-propyle, n-butyle, sec.-butyle, t-butyle, vinyle, allyle, propargyle, de même qu'un phényle et benzyle éventuellement substitués chacun une ou deux fois, de manière identique ou différente, par les substituants de phényle déjà cités pour $R^1$, et leurs sels d'addition d'acides et complexes de sels métalliques.

3. Procédé de fabrication d'azolyléthyloximinoalcoyléthers substitués de formule générale I:

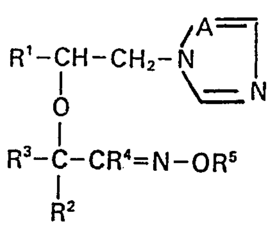

(I)

dans laquelle:

A représente un atome d'azote ou le groupe CH,

$R^1$ un phényle éventuellement substitué une ou deux fois, de manière identique ou différente, en citant comme substituants de l'halogène, alcoyle ayant 1 à 4 atomes de carbone, alcoxy ayant 1 à 2 atomes de carbone de même qu'un halogénoalcoyle et halogénoalcoxy ayant chacun 1 à 2 atomes de carbone et 1 à 5 atomes d'halogène identiques ou différents,

$R^2$ de l'hydrogène, un alcoyle ayant 1 à 4 atomes de carbone, de même qu'un phényle éventuellement substitué une ou deux fois, de manière identique ou différente, en envisageant comme substituants les substituants de phényle cités pour $R^1$,

$R^3$ de l'hydrogène, un alcoyle ayant 1 à 4 atomes de carbone, de même qu'un phényle éventuellement substitué une ou deux fois de manière identique ou différente, en envisageant comme substituants les substituants de phényle cités pour $R^1$,

$R^4$ de l'hydrogène, un alcoyle ayant 1 à 4 atomes de carbone, de même qu'un phényle éventuellement substitué une ou deux fois de manière identique ou différente, en envisageant comme substituants les substituants de phényle cités pour $R^1$,

$R^5$ de l'hydrogène, un alcoyle ayant 1 à 4 atomes de carbone, un alcényle et un alcinyle ayant chacun 2 à 4 atomes de carbone, de même qu'un phényle et phénylalcoyle éventuellement substitués chacun une ou deux fois de manière identique ou différente et ayant 1 à 2 atomes de carbone dans la portion alcoyle, en envisageant comme substituants sur les radicaux aromatiques chaque fois les substituants de phényle cités pour $R^1$,

caractérisé en ce qu'on fait réagir des azolyléthanols de formule générale II:

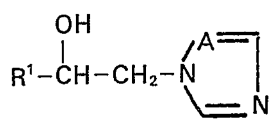

(II)

dans laquelle:

A et $R^1$ ont la signification indiquée plus haut, avec des dérivés d'oxime de formule générale III:

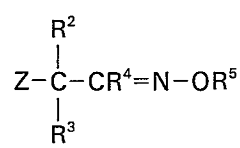

(III)

dans laquelle:

$R^2$, $R^3$, $R^4$ et $R^5$ ont la signification indiquée plus haut, et

Z représente de l'halogène, le radical méthylsulfonyloxy ou tolylsulfonyloxy, en présence d'une base et en présence d'un diluant organique, ou bien dans un système aquo-organique à deux phases en présence d'un catalyseur de transfert de phase, et en ce qu'éventuellement on fixe ensuite un acide ou un sel métallique.

4. Agents fongicides, caractérisés par une teneur en au moins un azolyléthyloximinoalcoyléther substitué de formule générale I selon les revendications 1 et 3.

5. Utilisation d'azolyléthyloximinoalcoyléthers substitués de formule générale I selon les revendications 1 et 3 pour combattre les champignons.